# DEMANDE DE BREVET EUROPEEN

(11) **EP 1 647 259 A1**
(43) Date de publication de la demande: **19.04.2006**
(21) Numéro de dépôt: 05292138.4
(22) Date de dépôt: 13.10.2005
(51) Int. Cl.: A61K 8/40, A61K 8/02, A61Q 5/06

(54) **Utilisation pour le traitement des matières kératiniques de compositions à base de monomères électrophiles et d'agents cristaux liquides**

(30) Priorité: 13.10.2004 FR 0410812
(71) Demandeur: L'OREAL, 75008 Paris (FR)
(72) Inventeur: Brun, Gaëlle, 75015 Paris (FR); Gourlaouen, Luc, 92600 Asnieres (FR)
(74) Mandataire: Casalonga, Axel

(57) **Abrégé**

La présente demande a pour objet l'utilisation pour le traitement des matières kératiniques, et notamment des fibres kératiniques telles que les cheveux, d'une composition comprenant, dans un milieu cosmétiquement acceptable, au moins un monomère électrophile, et au moins un agent cristaux liquides.

## Description

La présente invention concerne l'utilisation pour le traitement des matières kératiniques, et notamment des fibres kératiniques telles que les cheveux, de compositions à base de monomères polymérisables in situ, d'un milieu cosmétiquement acceptable, et d'agents cristaux liquides, ainsi que le procédé de traitement cosmétique correspondant.

Elle concerne aussi de nouvelles compositions à base de monomères électrophiles et d'agents cristaux liquides particuliers.

Par "matières kératiniques", on entend de préférence les fibres kératiniques, et encore de préférence les cheveux.

Pour qu'une coloration capillaire soit visible sur un fond de couleur foncée, sans que la coloration ne soit qu'un simple reflet, il est nécessaire d'éclaircir les matières kératiniques, et notamment les fibres. Dans les produits disponibles dans le commerce, cet éclaircissement passe par une étape de décoloration de la mélanine du cheveu utilisant des agents oxydants, ce qui abîme le cheveu.

Il est connu pour éclaircir la fibre de déposer un pigment à la surface de la fibre. En effet, le pigment étant en surface, il masque le fond de la fibre et est donc visible même sur un fond de couleur foncée. Toutefois, le dépôt d'un pigment à la surface de la fibre conduit à des dépôts non rémanents aux shampooings.

D'autre part, pour créer des couleurs, on utilise une gamme d'agents de coloration assez limitée, en particulier des pigments tels que des laques, des pigments minéraux ou des pigments nacrés. Les laques permettent d'obtenir des couleurs vives, mais pour la plupart sont instables à la lumière, à la température et au pH. Certaines présentent également l'inconvénient de tacher le cuir chevelu de manière disgracieuse après application, par dégorgement du colorant. Les pigments minéraux, en particulier les oxydes minéraux, sont au contraire, très stables, mais confèrent à la fibre des couleurs plutôt ternes et pâles. Pour obtenir des effets colorés, on peut employer des pigments nacrés de couleurs variées, mais jamais intenses, qui permettent d'obtenir des effets irisés mais le plus souvent assez faibles.

La demanderesse vient de découvrir de manière surprenante qu'il était possible d'obtenir de nouveaux effets de coloration en mettant en oeuvre un mélange de monomères électrophiles, tels qu'ils sont décrits dans la demande FR 2840208, d'un milieu cosmétiquement acceptable et d'agents cristaux liquides.

La demanderesse a plus particulièrement constaté qu'en appliquant sur les fibres une composition à base de tels monomères, d'un milieu cosmétiquement acceptable et d'agents cristaux liquides, on obtenait des couleurs distinctes selon l'incidence de la lumière et l'angle d'observation, avec de bonnes propriétés de brillance.

Elle a également constaté que la composition utilisée formait un gainage sur les fibres kératiniques, rémanent à plusieurs shampooings.

Par ailleurs, la demanderesse a constaté de façon surprenante que les cheveux restaient parfaitement individualisés et pouvaient être coiffés sans problème, et que le conditionnement et la brillance de la fibre étaient rémanents aux shampooings.

L'invention a donc pour premier objet l'utilisation pour le traitement des matières kératiniques, et notamment des fibres kératiniques telles que les cheveux, d'une composition comprenant, dans un milieu cosmétiquement acceptable, au moins un monomère électrophile, et au moins un agent cristaux liquides.

L'invention a également pour objet un procédé mettant en oeuvre une composition utilisée dans le cadre de ce traitement.

Elle a également pour objet une composition cosmétique comprenant, dans un milieu cosmétiquement acceptable, au moins un monomère électrophile, et au moins un agent cristaux liquides particulier.

Elle a enfin pour objet un kit comprenant une première composition contenant au moins un monomère électrophile et éventuellement au moins un inhibiteur de polymérisation anionique et/ou radicalaire, ainsi qu'une deuxième composition comprenant dans un milieu cosmétiquement acceptable au moins un agent cristaux liquides.

D'autres objets de l'invention apparaîtront à la lecture de la description et des exemples qui suivent.

Par monomère électrophile, on entend un monomère capable de polymériser par polymérisation anionique en présence d'un agent nucléophile tel que par exemple, les ions hydroxyles (OH-) contenus dans l'eau.

Par polymérisation anionique, on entend le mécanisme défini dans l'ouvrage "Advanced Organic Chemistry", Third Edition de Jerry March, pages 151 à 161.

Le ou les monomères électrophiles présents dans la composition de l'invention peuvent être choisis parmi :
- les dérivés benzylidene malononitrile (A), le 2-(4-chloro-benzylidene)-malononitrile (A1) le 2-cyano-3-phényl acrylate d'éthyle (B), le 2-cyano-3-(4-chloro-phényl) acrylate d'éthyle (B1) décrits dans Sayyah, *J. Polymer Research,* 2000, p97 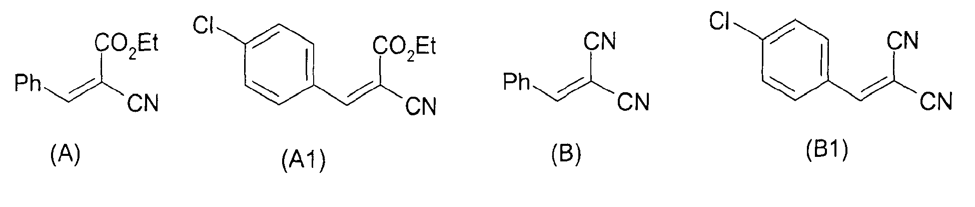
- les dérivés de méthylidenemalonates comme :
   - le 2-méthylene-malonate de diéthyle (C) par Hopff, *Makromoleculare Chemie,* 1961, p95, De Keyser, J. *Pharm. Sci,* 1991, p67 et Klemarczyk, *Polymer,* 1998, p173

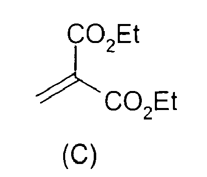

   - le 2-éthoxycarbonylméthyleneoxycarbonyl acrylate d'éthyle (D) par Breton, *Biomaterials,* 1998, p271 et Couvreur, *Pharmaceutical Research,* 1994, p1270. 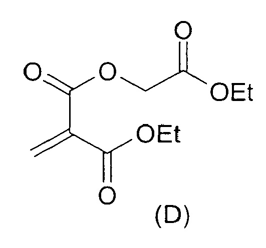
- les dérivés itaconate et itaconimide comme :
   - l'itaconate de diméthyle (E) par Bachrach, *European Polymer Journal,* 1976, p563 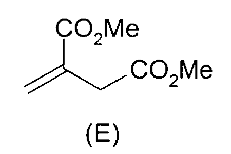
   - N-butyl itaconimide (F), N-(4-tolyl) itaconimide (G), N-(2-ethylphenyl) itaconimide (H), N-(2,6-diethylphenyl) itaconimide (I) par Wanatabe, *J.Polymer Science : Part A :Polymer chemistry,* 1994, p2073 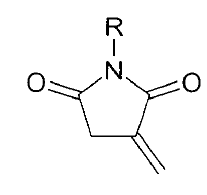 R= Bu (F), 4-tolyl (G), 2-ehylphenyl (H), 2,6-diethyphenyl (I)
- les dérivés α-(methylsulfonyl)acrylates de méthyle (K), α-(methylsulfonyl)acrylates d'éthyle (L), α-(tert-butylsulfonyl)acrylates de méthyle (M), α-(methylsulfonyl)acrylates de tert-butyle (N), α-( tert-butylsulfonyl)acrylates de tert-butyle (O), par Gipstein, *J. Org. Chem,* 1980, p 1486 et
- les dérivés 1,1-bis-(methylsulfonyl)ethylene (P), 1-acetyl-1-methyl sulfonyl ethylene (Q), α-(methylsulfonyl) vinyl sulfonate de methyle (R), α-methylsulfonylacrylonitrile (S) par Shearer, US patent US2748050. 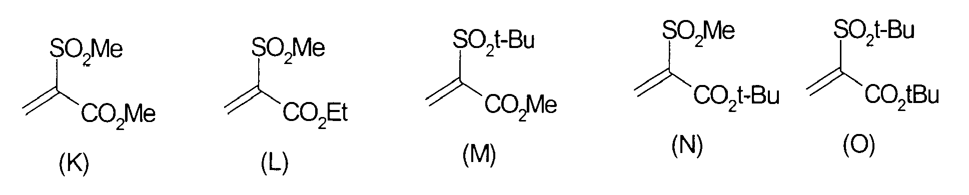 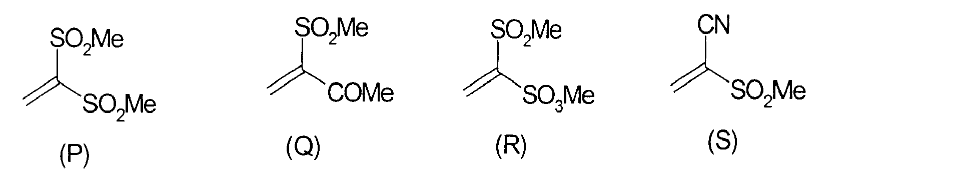
- les dérivés méthyl vinyl sulfone (T) et phényl vinyl sulfone (U) par Boor, *J.Polymer Science,* 1971, p249 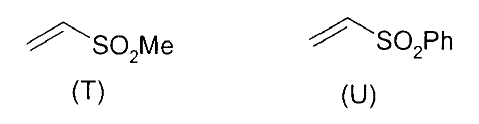
- le dérivé phényl vinyl sulfoxide (V) par Kanga, *Polymer preprints (ACS, Divison of Polymer Chemistry),* 1987, p322 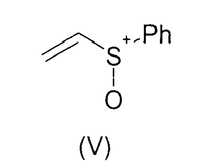
- le dérivé 3-methyl-N-(phenylsulfonyl)-1-aza-1,3-butadiene (W) par Bonner, *Polymer Bulletin,* 1992, p517 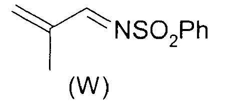
- les dérivés acrylates et acrylamides comme :
   - N-propyl-N-(3-triisopropoxysilylpropyl)acrylamide (X) et N-propyl-N-(3-triethoxysilylpropyl)acrylamide (Y) par Kobayashi, *Journal of Polymer Science, Part A: Polymer Chemistry,* 2005, p2754. 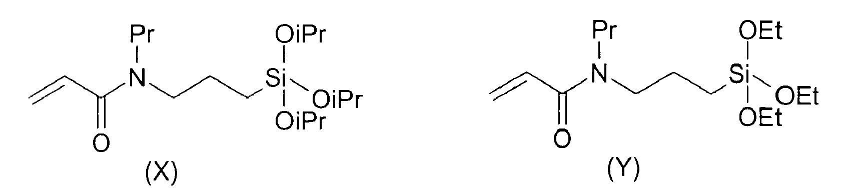
   - 2-hydroxyethyl acrylate (Z) et 2-hydroxyethyl méthacrylate (AA) par Rozenberg, *International Journal of Plastics Technology,* 2003, p17 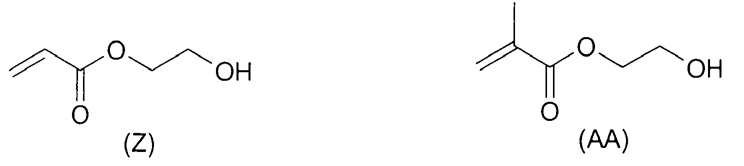
   - N-butyl acrylate (AB) par Schmitt, *Macromolecules,* 2001, p2115
   - Tert-butyl acrylate (AC) par Ishizone, *Macromolecules,* 1999, p955.

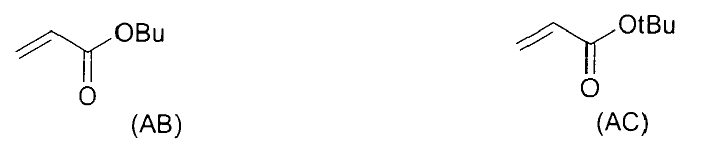

Le monomère électro-attracteur utile dans la présente invention peut être cyclique ou linéaire. Lorsqu'il est cyclique, le groupe éléctro-attracteur est de préférence exocyclique, c'est-à-dire qu'il ne fait pas partie intégrante de la structure cyclique du monomère.

Selon un mode de réalisation particulier, ces monomères présentent au moins deux groupes électro-attracteurs.

A titre d'exemple de monomères présentant au moins deux groupes électro-attracteurs, on peut citer les monomères de formule (I) : 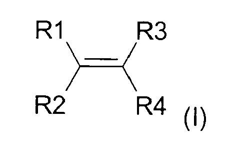 dans laquelle :
R₁ et R₂ désignent chacun, indépendamment l'un de l'autre, un groupe peu ou non électro-attracteur (peu ou non inductif-attracteur) tel que :
   - un atome d'hydrogène,
   - un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR,
   - OH, et les atomes d'halogène,
   - un résidu polyorganosiloxane modifié ou non,
   - un groupement polyoxyalkylène,
R₃ et R₄ désignent chacun, indépendamment l'un de l'autre, un groupe électro-attracteur (ou inductif-attacteur) choisi de préférence parmi les groupements -N(R)₃⁺, -S(R)₂⁺, -SH₂⁺, -NH₃⁺, -NO₂, -SO₂R, -C≡N, -COOH, -COOR, -COSR, -CONH₂, -CONHR, -F, -Cl, -Br, -I, - OR, -COR, -SH, -SR, -OH, les groupes alcényle linéaires ou ramifiés, les groupes alcynyle linéaires ou ramifiés, les groupements mono- ou polyfluoroalkyle en C₁-C₄, les groupements aryle tels que phényle, ou les groupements aryloxy tels que phénoxyloxy,
R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', - COOR', -COR', -SH, -SR', -OH, les atomes d'halogène, ou un résidu de polymère, R' désignant un radical alkyle en C₁-C₁₀, ce polymère pouvant être obtenu par polymérisation radicalaire, par polycondensation ou par ouverture de cycle.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, mieux encore de 1 à 10 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et/ou siloxy et/ou silanol et/ou amine et/ou imine et/ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH₂)n-(CF₂)m-CF₃ ou -(CH₂)n-(CF₂)m-CHF₂ avec n=1 à 20 et m= 1 à 20.

Les substituants R1 à R4 peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoïques, quinoniques, méthiniques, cyanométhiniques et triarylméthanes.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et les groupements de type esters gras.

Parmi les monomères précédemment cités, sont préférés les monomères de la famille des cyanoacrylates et leurs dérivés de formule (II) : 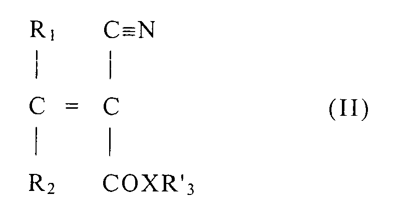 X désignant NH,S,O,
R₁ et R₂ ayant les mêmes significations que précédemment,
R'₃ pouvant désigner un atome d'hydrogène ou un radical R tel que défini pour la formule (I).

De préférence, X désigne O.

A titre de composés de formule (B), on peut citer les monomères :
a) appartenant à la famille des 2-cyanoacrylates de polyfluoroalkyle en C₁-C₂₀ tels que :
   l'ester 2,2,3,3-tétrafluoropropylique de l'acide 2-cyano-2-propénoïque de formule : 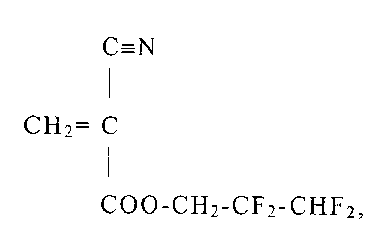
   ou encore l'ester 2,2,2-trifluoroéthylique de l'acide 2-cyano-2-propénoïque de formule : 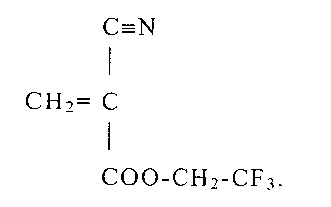
b) les cyanoacrylates d'alkyle en C₁-C₁₀ ou d'(alcoxy en C₁₋C₄)(alkyle en C₁-C₁₀).
   On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

Dans le cadre de l'invention, on préfère utiliser les monomères b).

Les monomères les plus particulièrement préférés sont ceux de formule III et leurs mélanges : 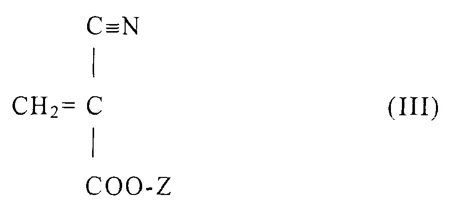 dans laquelle : Z=-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Par milieu cosmétiquement acceptable, on entend un milieu compatible avec les matières kératiniques telles que les cheveux.

Le milieu cosmétiquement acceptable est de préférence anhydre. On entend par « milieu anhydre », un milieu contenant moins de 1 % en poids d'eau par rapport au poids total de la composition.

Le milieu cosmétiquement acceptable est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ; les cires ; ou encore des composés organiques tels que des alcanes en C₅-C₁₀, l'acétone, la méthyléthylcétone, les esters d'acides en C₁-C₂₀ et d'alcools en C₁-C₈ tels que l'acétate de méthyle, l'acétate de butyle, l'acétate d'éthyle et le myristate d'isopropyle, le diméthoxyéthane, le diéthoxyéthane, les alcools gras en C₁₀-C₃₀ tels que l'alcool laurique, l'alcool cétylique, l'alcool stéarylique, l'alcool béhénylique ; les acides gras en C₁₀-C₃₀ tels que l'acide laurique, l'acide stéarique ; les amides gras en C₁₀-C₃₀ tels que la diéthanolamide laurique, les esters d'alcools gras en C₁₀-C₃₀ tels que les benzoates d'alcool gras en C₁₀-C₃₀ et leurs mélanges.

De préférence, les composés organiques sont choisis parmi les composés liquides à la température de 25°C et sous 10⁵ Pa (760mm de Hg).

Les compositions mises en oeuvre conformément à l'invention ont généralement une concentration en monomère électrophile selon l'invention comprise entre 0,001 et 80 % en poids, et plus particulièrement entre 0,1 et 40 % et encore plus préférentiellement entre 1 et 20 % en poids par rapport au poids total de la composition.

On peut également introduire dans les compositions, des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges. Les groupements alkyle désignent de préférence des groupements ayant 1 à 6 atomes de carbone.

On peut aussi utiliser des acides organiques ou minéraux, ces derniers ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique.

La quantité d'inhibiteur peut aller de 10 ppm à 20%, et plus préférentiellement de 10 ppm à 5% et encore plus préférentiellement de 10 ppm à 1 % en poids par rapport au poids total de la composition.

Les compositions conformes à l'invention peuvent également contenir au moins un agent utilisé habituellement en cosmétique, tel que, par exemple, des agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, cationiques, non ioniques ou amphotères, des polymères fixants ou non, des polyols, des protéines, des vitamines, des colorants directs ou d'oxydation, et des agents nacrants, des gaz propulseurs, des épaississants minéraux ou organiques tels que le benzylidène sorbitol, les N acylaminoacides. Ces agents peuvent être éventuellement encapsulés. La capsule peut être de type polycyanoacrylate.

On entend par agents cristaux liquides des composés générant un état mésomorphe, c'est-à-dire un état pour lequel la fusion des cristaux permet d'obtenir des liquides possédant des propriétés optiques comparables à celles de certains cristaux. Ces composés sont plus précisément définis dans le chapitre Liquid crystals dans l'encyclopédie Ullmann.

Ces agents cristaux liquides sont en particulier décrits dans les brevets ou demandes de brevet EP 545 409, WO 94109086, EP 709 445, GB 2 282 145, GB 2 276 883, WO 95132247, WO 95132248, EP 686 674, EP 711 780.

Plus particulièrement, les composés générant un état mésomorphe peuvent être :
o Les composés à fonction cholestérique, dont la structure est la suivante : 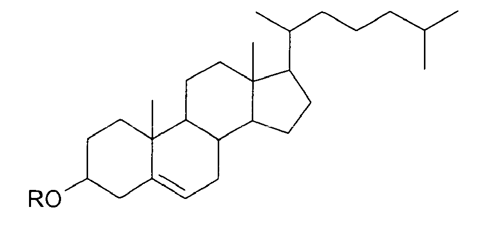
R est un groupement alkyl, alkylcarbonyl comprenant de 1 à 30 atomes de carbone substitué ou non par des groupements cycliques, aromatiques, halogènes, ramifié ou non.

A titre non limitatif, on peut citer comme agent cristaux liquides répondant à cette définition : le cholesterol erucyl carbonate, le choleterol methyl carbonate, le cholesterol oleyl carbonate, le cholesterol para-nonyl phneyl carbonate, le cholesterol phenyl carbonate, la cholesterol acetate, le cholesterol benzoate, le cholesterol butyrate, le cholesterol isobutyrate, le cholesterol chloride, le cholesterol chloroacetate, le cholesterol cinnamate, le cholesterol crotanoate, le cholesterol decanoate, le cholesterol erucate, le cholesterol heptanoate, le cholesterol hexanoate, le cholesterol myristate, le cholesterol nonaoate, le cholesterol octanoate, le cholesterol oleate, le cholesterolpropionate, le cholesterol valerate, le dicholesteryl carbonate.
o Les composés définis par la formule suivante :

   A₁^{5'}- [(X¹)ₐ- (A¹)_{b}- (A²)_{c}]_{d}-Zₑ-[(X²)_{f}-(A³)_{g}-(A⁴)ₕ]ᵢ-Aₖ⁵

   dans laquelle
   X¹ et X² représentent indépendamment des radicaux divalents - O-, -COO-, -CONH-, -CO-, -S-, -C≡C-, -CH=CH-, -CH₂-CH₂,-, -CH=N-, -N=N- ou -N=N(O)-, -CH=N-N=CH-, -CH=CH-COO-, -OCO-CH=CH-
   A¹, A², A³ et A⁴, représentent indépendamment des radicaux divalents 1,4-phénylène, 1,4-cyclohexylène, arylènes, hétéroarylènes, hétérocycloalkylènes, cycloalkylènes éventuellement substitués,
   Z représente indépendamment des radicaux divalents à tétravalents, benzène-1,4 cyclohexane ou benzène-1,3-cyclopentane,
   A⁵ et A^{5'} représentent indépendamment un radical alkyle, alkoxy ou cycloalkyle, saturé ou insaturé, ayant 1 à 16 atomes de carbone, un radical stéroïdien, un halogène, un atome d'hydrogène, un radical hydroxyle, nitrile ou trialkylsilyloxy.
   a, b, c, d, f, g, h, i k et 1 représentent indépendamment un entier allant de 0 à 3,
   e représente 0 ou 1,
   avec la somme a+b+c+d+e+f+g+h+i+k étant supérieure ou égale à 2, et la somme d+i étant inférieure ou égale à 4, étant entendu que ce groupe ne comprend pas de radical peroxyde.

On peut citer à titre non limitatif comme agents cristaux liquides répondant à cette formule, les composés suivants :

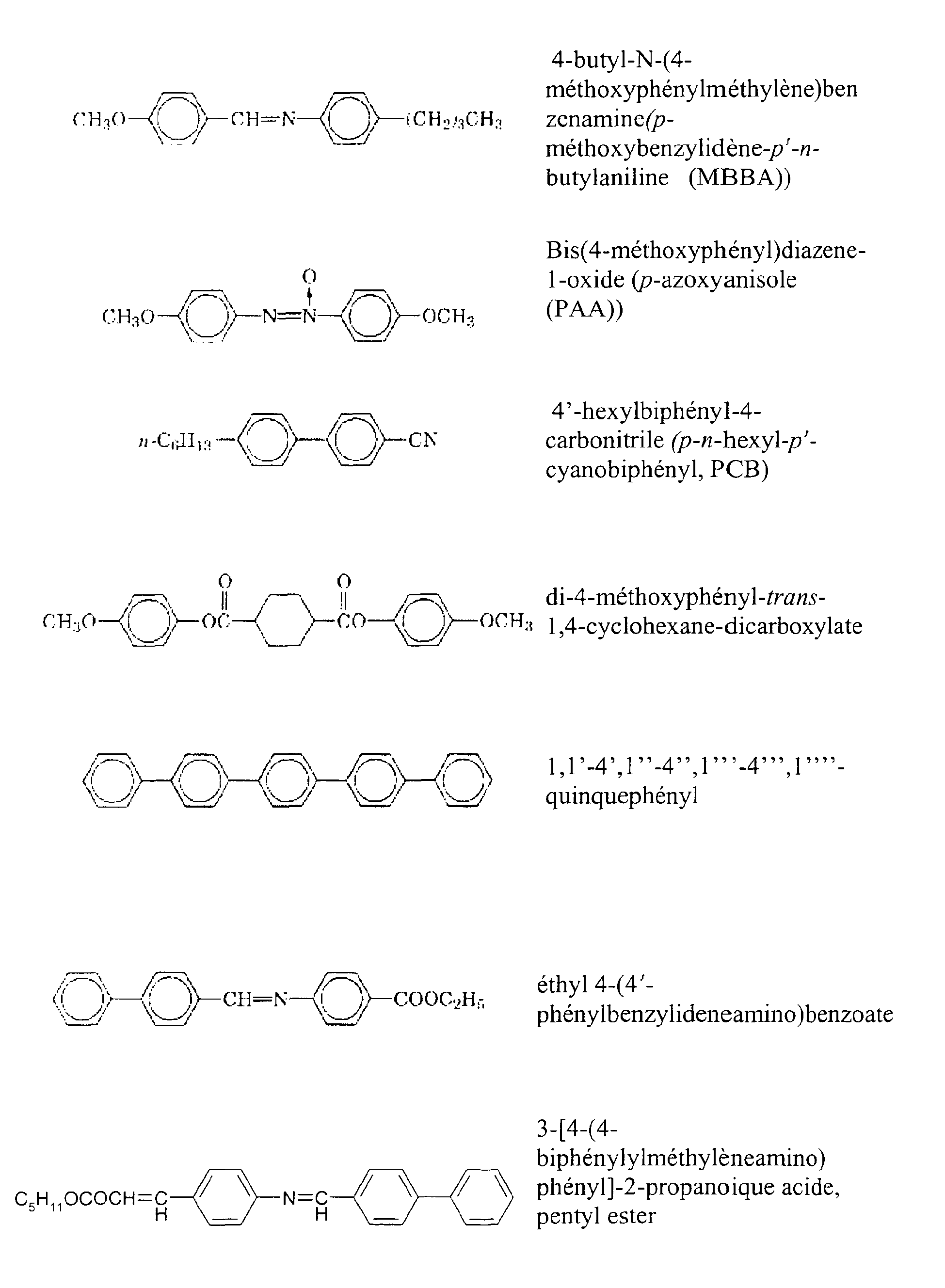

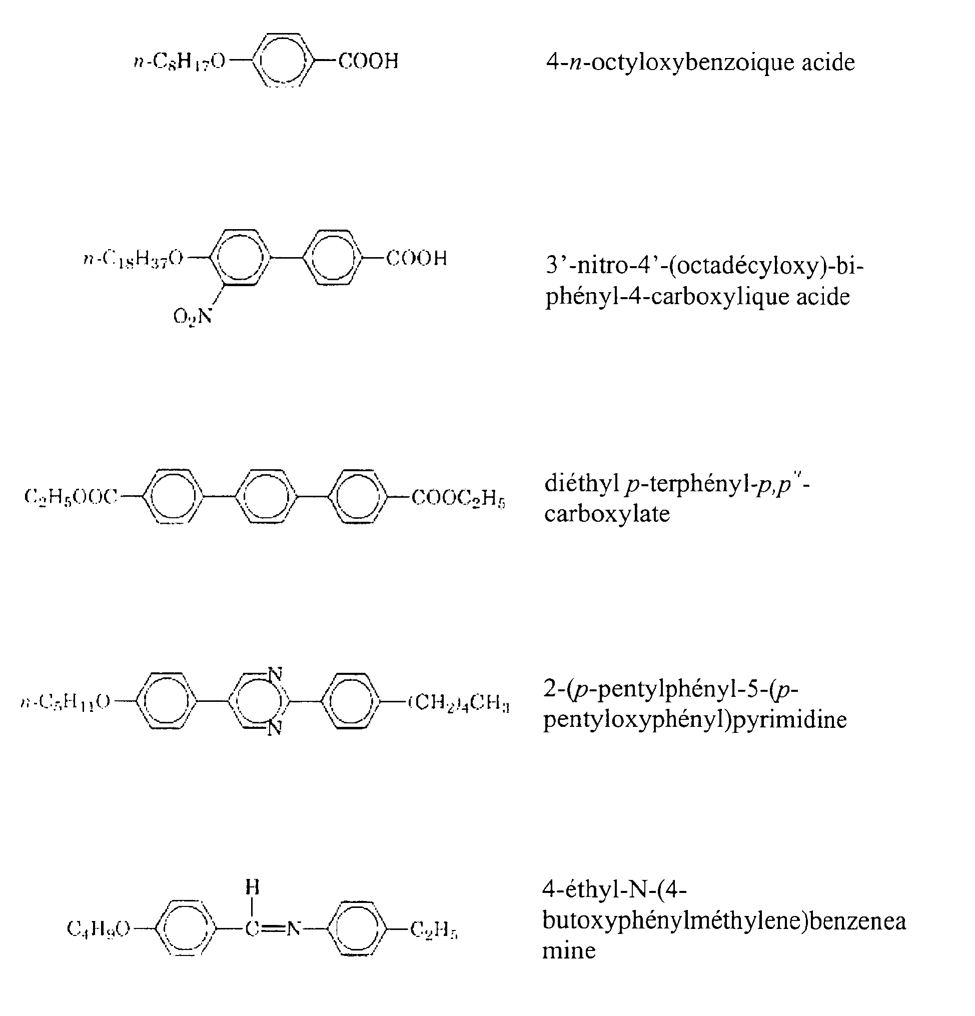

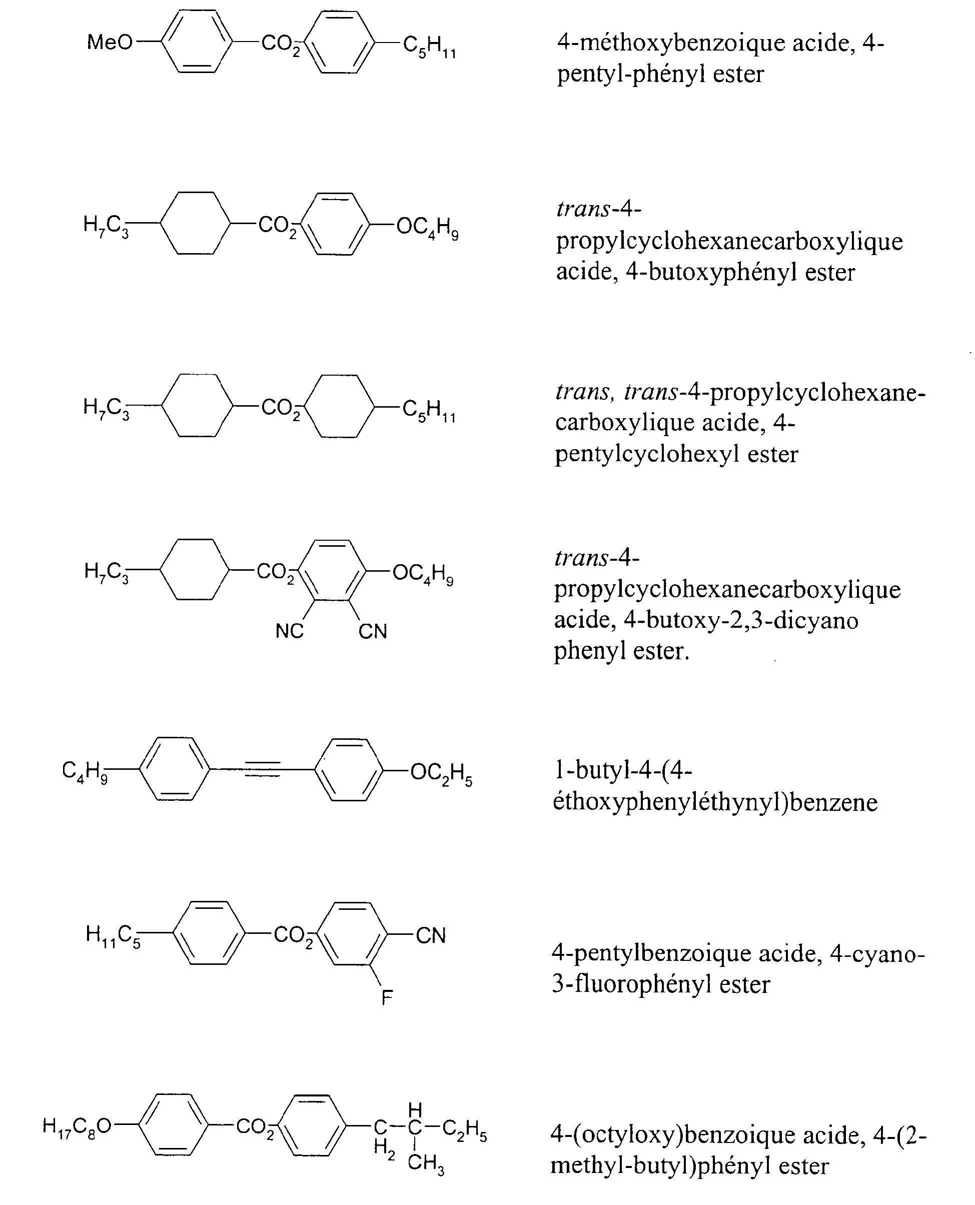

o Un polymère obtenu par la polymérisation de monomères mésogéniques, tels qu'ils peuvent être décrits par exemple dans les brevets EP 1302524, EP 1304161, EP 1422283, WO 02/086609, GB 2337753. Un monomère mésogénique est un monomère qui donne des propriétés de cristaux liquides aux polymères obtenus à partir de ces monomères (tel que défini dans l'IUPAC, 2è édition de 1997). Ces monomères ont la structure suivante :

   Y1-B1-M-R

   Dans laquelle :
   Y1 représente un groupe polymérisable choisi parmi les groupements acrylate ou méthacrylate, un groupement époxy, isocyanante, hydroxy, vinyléther (-O-CH=CH2), vinylester (-CO-OC=CH2), styryl, trialkoxysiloxy
   B1 représente un groupement de formule -CnH2n-, dans lequel n est un nombre entier allant de 0 à 20 et un ou plusieurs groupes méthylènes dudit groupement -CnH2n-pouvant être substitués par un atome d'halogène ou être remplacé (lorsque les méthylènes ne sont pas adjacents) par un ou plusieurs groupes suivants : -O-, -NH-, -OCO-, - OCO-O-, -S-CO--COO-, -CONH-, -CO-, -S-, -C≡C-, -CH=CH-.
   M représente un groupement de formule :

      -[(X¹)ₐ- (A¹)_{b}- (A²)_{c}]_{d}-Zₑ-[(X²)_{f}-(A³)_{g}-(A⁴)ₕ]ᵢ-

      dans laquelle
      X¹ et X² représentent indépendamment des radicaux divalents - O-, -COO-, -CONH-, -CO-, -S-, -C≡C-, -CH=CH-, -CH₂-CH₂,-, -CH=N-, -N=N- ou -N=N(O)-, -CH=N-N=CH-, -CH=CH-COO-, -OCO-CH=CH-
      A¹, A², A³ et A⁴, représentent indépendamment des radicaux divalents 1,4-phénylène, 1,4-cyclohexylène, arylènes, hétéroarylènes, cycloalkylènes éventuellement substitués,hétérocycloalkylènes
      Z représente indépendamment des radicaux divalents à tétravalents, benzène-1,4 cyclohexane ou benzène-1,3-cyclopentane, Z peut aussi représenter un groupement chiral divalent comprenant au moins 4 atomes de carbone, notamment de 4 à 20 atomes de carbone et mieux de 4 à 10 atomes de carbone. Le groupement chiral divalent comprenant au moins un carbone asymétrique, notamment un ou deux carbone asymétriques et en particulier deux carbones asymétriques. Les groupements chiraux sont en particulier ceux décrits dans le brevet WO 98/00428. Le groupement chiral est en particulier issu du groupe des dianhydrohexites, hexoses, pentoses, les dérivés de binaphtyle (groupements binaphtyl), les dérivés biphényles (groupements biphényle), les dérivés d'acides tartriques ou des glycols optiquement actifs. De préférence, lorsque le groupement Z est chiral, il désigne un groupement chiral à deux liaisons, issu du groupement dianhydrohexite et en particulier un radical de formule : 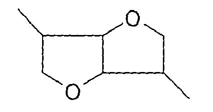 a, b, c, f, g et h représentent indépendamment un entier allant de 0 à 3,
      e représente 0 ou 1,
      avec la somme a+b+c+d+e+f+g+h+i+k étant supérieure ou égale à 2,

R représente indépendamment un radical alkyle, alkoxy ou cycloalkyle, saturé ou insaturé, ayant 1 à 16 atomes de carbone, un radical stéroïdien, un halogène, un atome d'hydrogène, un radical hydroxyle, nitrile, trialkylsilyloxy, un radical cholestéryl. R peut aussi représenter une structure type B2-Y2, les définitions de B2 et Y2 étant identiques respectivement à celles de B1 et Y1. Les composés Y1 et Y2, B1 et B2 pouvant être identiques ou différents.

A titre d'exemple, on peut citer les monomères suivants :

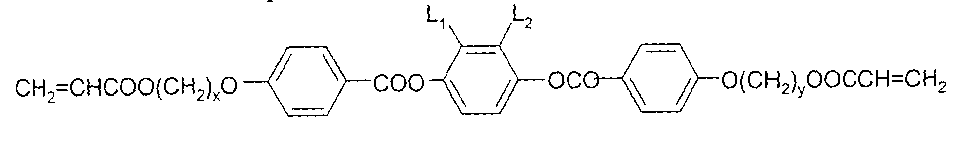

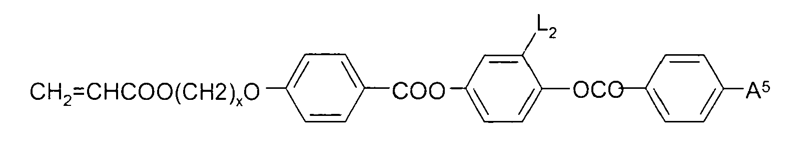

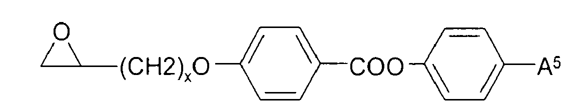

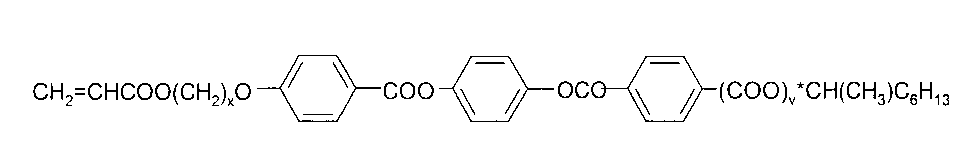

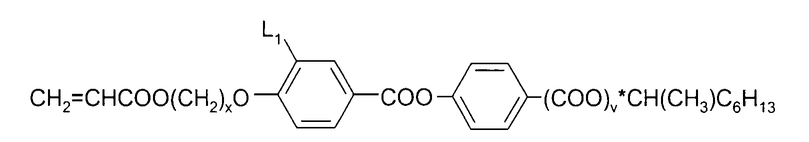

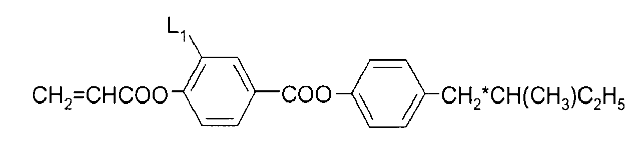

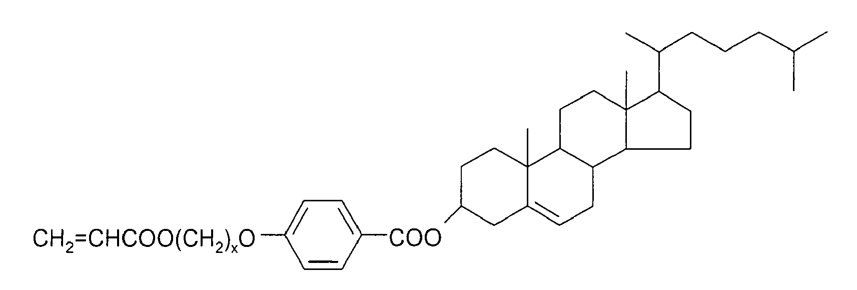

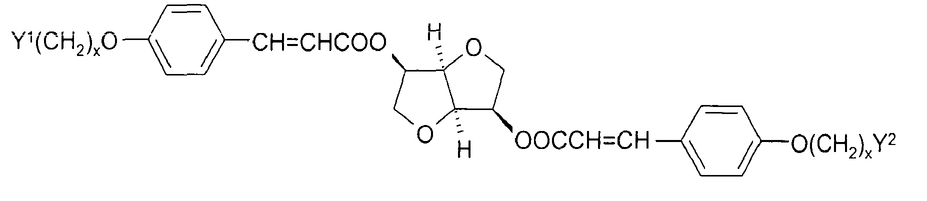

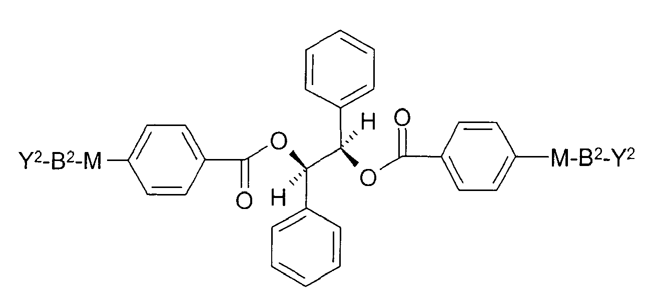

L1 et L2 étant indépendamment l'un de l'autre H, F, Cl, CN, un groupement alkyl, alkoxy, alkylcarbonyl, alkyl halogéné, alkoxycarbonyl ou alkoxycabonyloxy avec de 1 à 7 atomes de carbone. x est un entier allant de 0 à 20.

Dans les compositions selon l'invention, l'agent cristaux liquides est différent des agents cristaux liquides cholestériques.

Le polymère cristaux liquides obtenu peut être obtenu à partir de la polymérisation d'un monomère mésogénique seul ou avec un ou plusieurs autres monomères qui peuvent être mésogéniques ou non. Si le polymère cristaux liquides est obtenu à partir de la polymérisation d'un ou plusieurs monomères mésogéniques, alors il est de préférence obtenu avec au moins un composé mésogénique polymérisable ayant un groupe fonctionnel polymérisable et au moins un composé mésogénique polymérisable ayant deux ou plus groupes fonctionnels polymérisables. De manière encore plus préférentielle, la polymérisation n'est effectuée qu'avec des composés mésogéniques polymérisables ayant deux ou plus groupes fonctionnels polymérisables. Dans ce cas, un réseau de polymère est obtenu.

De préférence, le polymère final est obtenu par la polymérisation d'un monomère mésogénique polymérisable chiral et d'un monomère mésogénique polymérisable non chiral.

Les composés mésogéniques polymérisables comprenant des fonctions mono, di ou multiréactives peuvent être préparés selon des méthodes qui sont décrites par exemple dans les ouvrages classiques de chimie organique comme par exemple, Houben-Weyl, Methoden der organischen Chemie, Thieme-verlag, Stuttgart. D'autres exemples sont décrits dans les brevets WO 93/22397, EP 0261712, DE 19504224, DE 4408171, DE 4405316, US5362315, US 5807497.

Les conditions de polymérisation sont celles classiquement utilisées pour les fonctions réactives portées par les monomères. Le mode de polymérisation est décrit par exemple dans le brevet EP1302524.

Une forme particulièrement préférée d'agent cristaux liquides conforme à l'invention consiste en des polymères tels qu'ils sont décrits dans la demande de brevet EP 1046692. Comme particules de polymères à cristaux liquides répondant à la définition précédente ont peut citer celles connues sous le nom CTFA Polyacrylate-4 et vendues sous les dénominations « Helicone® HC Sapphire », « Helicone® HC Scarabeus », « Helicone® HC Jade », « Helicone® HC Maple », « Helicone® HC S Sapphire », « Helicone® HC S Scarabeus », « Helicone® HC S Jade », « Helicone® HC S Maple » par la société Wacker.

Une autre forme particulièrement préférée d'agent cristaux liquides conforme à l'invention consiste en des polyorganosiloxanes cycliques greffés par des groupements cholestériques et biphényliques. Ils sont décrits notamment dans l'article de H.J. EBERLE, A MILLER, F. H. KREUZER Liquid Crystals, 1989, Vol 5, N°3, 907-916, dans l'article de J. PINSL, CHR. BRAÜCHIE, F. H. KREUZER, Journal of Molecular Electronics, Vol 39-13 (1987) et le brevet USP 4 410 570.

Ils sont choisis encore plus particulièrement parmi les cyclométhicones greffées par des groupements cholestériques et biphényliques. Ces composés sont cités dans la demande de brevet EP815826.

A titre d'exemples d'agent cristaux liquides répondant à cette définition, on peut citer en particulier les « pigments CL » commercialisés par la société WACKER sous les dénominations SLM 41101 (BLEU/VERT), SLM 41102 (ROUGE/OR) et SLM 41103 (JAUNE/VERT).

Ces agents cristaux liquides peuvent être employés seuls et/ou enrobés sur des supports inertes tels que des micas, et/ou associés avec d'autres agents non cristaux liquides.

Afin de modifier le reflet obtenu sur le cheveu, il est par exemple possible d'appliquer en plus de l'agent cristaux liquides un agent de coloration.

Par agent de coloration, on entend des colorants solubles non cristaux liquides et/ou des pigments non cristaux liquides usuels employés pour la coloration capillaire.

Il peut s'agir notamment de pigments résultant de la polymérisation oxydante d'un dérivé indolique tel que décrit dans la demande de brevet FR 2 679 771.

II peut s'agir également de tous les pigments organiques ou minéraux qui ne résultent pas de la polymérisation oxydante de composés indoliques, cosmétiquement ou dermatologiquement acceptables.

Ils peuvent se présenter sous forme de poudre ou de pâte pigmentaire.

Parmi les pigments minéraux, on peut citer à titre d'exemple le dioxyde de titane (rutile ou anastase) éventuellement traité en surface et codifié dans le Colour Index sous la référence CI77891 ; les oxydes de fer noir, jaune rouge et 5 brun, codifiés sous les références C177499, 77492, 77491 ; le violet de manganèse (C177742) ; le bleu outremer (C177007) ; l'oxyde de chrome hydraté (C177289) ; le bleu ferrique (CI7751 O).

Parmi les pigments organiques, on peut citer à titre d'exemple, le pigment YELLOW 3 vendu notamment sous la dénomination commerciale "JAUNE COVANOR W 1603" par la société WACKER (CI 17710), le "D & C RED n° 19" (CI 45170), le "D & C RED n° 9 (CI 15585), le "D & C RED n° 21" (CI 45380), le "D & C ORANGE n° 4" (CI 15510), le "D & C ORANGE n° 5" (CI 5370), le "D & C RED n° 27" (CI 45410), le "D & C RED n"°3 (CI 15630), le "D &C RED n° 7" (CI 15850-1), le "D & C RED n° 6 (CI 15850-2), le "D & C YELLOW n° 5" (CI 19140), le "D & C RED n° 36" (CI 12085), le "D & C ORANGE n° 10 " (CI 45425), le "D & C YELLOW n° 6" (CI 15985), le "D & C RED n° 30" (CI 73360), le "D & C RED n° 3" (CI 45430), le noir de carbone (CI 77266), et les laques à base de carmin de cochenille (CI 75470).

On peut également utiliser des pigments nacrés qui peuvent être notamment choisis parmi les pigments nacrés blancs tels que le mica recouvert d'oxyde de titane, l'oxyde de bismuth ; les pigments nacrés colorés tels que le mica titane avec des oxydes de fer, le mica titane avec du bleu ferrique ou de l'oxyde de chrome, le mica titane avec un pigment organique de type précipité, ainsi que ceux à base d'oxychlorure de bismuth.

On peut utiliser plus particulièrement les pâtes pigmentaires de pigment organique telles que les produits vendus par la société HOECHST sous le nom :
JAUNE COSMENYL 10G : Pigment YELLOW 3 (CI 11710)
JAUNE COSMENYL G : Pigment YELLOW 1 (CI 11680)
ORANGE COSMENYL GR: Pigment ORANGE 43 (CI 71 105)
ROUGE COSMENYL R : Pigment RED 4 (CI 12085)
CARMIN COSMENYL FB : Pigment RED 5 (CI 12490)
VIOLET COSMENYL RL : Pigment VIOLET 23 (CI 51319)
BLEU COSMENYL A2R : Pigment BLUE 15.1 (CI 74260)
VERT COSMENYL GG : Pigment GREEN 7 (CI 74260)
NOIR COSMENYL R : Pigment BLACK 7 (CI 77266)

On peut aussi utiliser des colorants solubles, notamment des colorants appartenant à la familles des nitrés benzéniques ou hétérocycliques, des quinones (anthraquinones, naphtoquinones, benzoquinones), des azoïques, etc.

Que ce soit pour l'agent cristaux liquides ou pour l'agent de coloration, l'agent peut être présent à raison de 0,05 à 40 % du poids total de la composition, de préférence de 0,1 à 35 % et mieux de 0.25 à 25 %.

Lorsque la composition comprend des agents de coloration, le rapport pondéral agents cristaux liquides / agents de coloration est avantageusement compris entre 1/20 et 20/1, de préférence compris entre 1/10 et 10/1, plus préférentiellement compris entre 1/5 et 5/1.

Le procédé de traitement des cheveux conforme à l'invention consiste à appliquer la composition décrite ci-dessus sur les matières kératiniques, et en particulier en présence d'un agent nucléophile avec ou sans chauffage.

De préférence, cet agent nucléophile est l'eau. Cette eau peut être apportée par une humidification préalable.

Il est également possible, afin de moduler la cinétique de réaction, de préalablement humidifier les matières kératiniques à l'aide d'une solution aqueuse dont le pH a été ajusté à l'aide d'une base, d'un acide ou d'un mélange acide/base. L'acide et/ou la base peuvent être inorganique ou organique.

Ces deux opérations peuvent aussi être effectuées après application de la composition.

Il est également possible de moduler la cinétique de polymérisation par voie anionique en pré-imprégnant les matières kératiniques à l'aide d'un agent nucléophile. L'agent nucléophile peut être utilisé pur, en solution, sous forme d'une émulsion, ou être encapsulé.

Les agents nucléophiles susceptibles d'initier la polymérisation anionique sont des systèmes connus en eux-mêmes, capables de générer un carbanion au contact d'un agent nucléophile, tels que les ions hydroxyles contenus dans l'eau. On entend par « carbanion », les espèces chimiques définies dans « Advanced Organic Chemistry, Third Edition », de Jerry March, page 141.

Les agents nucléophiles peuvent être constitués par un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PhNH⁻, pyridine, ArS⁻, R-C≡C⁻, RS⁻, SH, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃-, ClO₄⁻ et H₂O, Ph représentant le groupe phényle ; Ar représentant un groupe aryle et R représentant un groupe alkyle en C₁-C₁₀.

De préférence, l'agent nucléophile est l'eau. Cette eau peut être apportée par une humidification préalable.

Il est également possible, afin de moduler la cinétique de réaction, de préalablement humidifier la fibre à l'aide d'une solution aqueuse dont le pH a été ajusté à l'aide d'une base, d'un acide ou d'un mélange acide/base. L'acide et/ou la base peuvent être inorganiques ou organiques.

Il est également possible de moduler la cinétique de polymérisation par voie anionique en pré-imprégnant la fibre à l'aide d'un agent nucléophile autre que l'eau. L'agent nucléophile peut être utilisé pur, en solution, sous forme d'une émulsion ou être encapsulé.

Pour moduler la cinétique de polymérisation anionique, on peut également augmenter la nucléophilie de la fibre par transformation chimique de la matière kératinique.

A titre d'exemple, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants:
- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium,
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

Pour moduler la cinétique de polymérisation par voie anionique, et plus précisément réduire la vitesse de polymérisation des monomères de l'invention, il est possible d'augmenter la viscosité de la composition. Pour ce faire, on peut ajouter à la composition de l'invention un ou des polymères ne présentant pas de réactivité sur les monomères conformes à l'invention. Dans ce cadre, on peut citer de façon non exhaustive le poly(méthacrylate de méthyle) (PMMA) ou encore les copolymères à base de cyanoacrylate tels qu'ils sont décrits dans le brevet US 6,224,622.

Afin d'améliorer entre autres l'adhésion du poly(cyanoacrylate) formé in situ, on peut pré-traiter la fibre avec tous types de polymères, ou réaliser un traitement capillaire avant application de la composition de l'invention, comme une coloration directe ou d'oxydation, une permanente ou encore un défrisage.

L'application des compositions peut être suivie ou non d'un rinçage. Ces compositions peuvent se présenter sous des formes diverses, telles que de lotion, de spray, de mousse.

Le mode d'application peut être en une seule étape ou bien être divisé en étapes successives.

Le procédé peut comprendre une étape d'application sur les matières kératiniques d'au moins un agent cristaux liquides et une étape d'application sur les matières kératiniques d'au moins un monomère électrophile, l'ordre des étapes étant indifférent.

Dans un mode de réalisation particulier, l'application du ou des agents cristaux liquides est réalisée avant l'application du ou des monomères électrophiles.

Si le procédé comporte plusieurs étapes d'application de compositions, ce peuvent être les suivantes :
1) application sur les cheveux de l'agent cristaux liquides, et éventuellement de l'agent de coloration non cristaux liquides, présents en solution aqueuse à raison de 0,05 à 40 %, de préférence de 0,1 à 35 % et mieux de 0,25 à 25 % en poids.
2) application sur les cheveux humidifiés du monomère, présent en solution à une concentration comprise entre 0,05 et 30 % en poids, plus préférentiellement comprises entre 0,01 et 50 % en poids, et plus préférentiellement entre 0,1 et 20 % en poids.

En plus de ces composés, chaque composition peut contenir des additifs cosmétiques conventionnels. L'ordre des deux étapes peut être inversé. La première étape peut être précédée de l'application d'un produit cosmétique, notamment de soin. De même, la dernière étape peut être succédée de l'application d'un produit cosmétique. Chaque étape peut être interrompue par un rinçage, un séchage. Le séchage peut être effectué au casque, au sèche cheveux et/ou au fer à lisser.

Selon les mêmes procédés, il est possible de réaliser des superpositions multiples de couches pour atteindre le type de dépôt souhaité (en termes de nature chimique, résistance mécanique, épaisseur, aspect, toucher...).

Selon la présente invention, les monomères sont de préférence choisis parmi les monomères capables de polymériser sur les fibres kératiniques dans des conditions cosmétiquement acceptables. En particulier, la polymérisation du monomère s'effectue de préférence à une température inférieure ou égale à 80°C, de préférence entre 10 et 80°C, de préférence de 20 à 80°C, ce qui n'empêche pas de terminer l'application par un séchage au casque, un brushing ou passage au fer plat ou à friser.

L'invention a également pour objet un kit comprenant une première composition contenant au moins un monomère électrophile et éventuellement au moins un inhibiteur de polymérisation anionique et/ou radicalaire, ainsi qu'une deuxième composition comprenant dans un milieu cosmétiquement acceptable au moins un agent cristaux liquides.

Les exemples qui suivent sont destinés à illustrer l'invention, sans toutefois présenter un caractère limitatif.

### Exemples

Des essais ont été réalisés en utilisant les composés suivants :
- *monomère capable de polymériser par voie anionique en présence d'un agent nucléophile* : 2-cyanoacrylate de n-octyle stabilisé avec 1% d'acide phosphorique
- *agent de coloration cristaux liquides 1* : Pigment CL SLM 41102 commercialisé par Wacker
- *agent de coloration cristaux liquides 2 :* Pigment Helicone HC Scarabeus commercialisé par Wacker
- *milieu cosmétiquement acceptable :*
   50% de mélange poly dimethylsiloxane alpha-omega dihydroxyle / cyclopenta dimethylsiloxane (14.7/85.3) commercialisé par Dow Corning sous le nom DC 1501 Fluid, et
   50% de cyclopentadimethylsiloxane commercialisé par Dow Corning sous le nom DC 245 Fluid

On travaille avec une mèche de 1g de cheveux naturels de hauteur de ton 4 ce qui correspond à une nuance naturelle châtain selon la classification des nuances naturelles décrite dans "Science des Traitements Capillaires" de C. ZVIAK, Ed. Masson 1988, p. 278.

### Exemple 1

Une solution aqueuse est préparée avec 10% d'agent cristaux liquides 1. 0.5g de cette solution aqueuse est appliqué sur cheveux propres et secs. La mèche est ensuite séchée au casque.

La mèche est humidifiée avec 0.5g d'eau. 0.5g d'une solution du milieu cosmétique à 10% en monomère est ensuite appliqué sur la mèche.

Après 10 minutes de pose la mèche est séchée pendant 2 minutes au sèche-cheveux.

La mèche obtenue possède un reflet irisé esthétique bronze ou vert selon l'angle d'observation. L'effet observé est rémanent à au moins six shampooings.

### Le mode d'application peut également être le suivant :

Une solution dans le milieu cosmétique est préparée avec 10% d'agent de coloration cristaux liquides 1. Le monomère est ajouté de manière à ce qu'il ait une concentration finale en monomère de 10%.

0.5g de cette solution est appliqué sur la mèche humidifiée avec 0.5g d'eau.

Après 10 minutes de pose, la mèche est séchée pendant 2 minutes au sèche-cheveux.

### Exemple 2

Une solution aqueuse est préparée avec 10% d'agent cristaux liquides 2. 0.5g de cette solution aqueuse est appliqué sur cheveux propres et secs. La mèche est ensuite séchée au casque.

La mèche est humidifiée avec 0.5g d'eau. 0.5g d'une solution du milieu cosmétique à 10% en monomère est ensuite appliqué sur la mèche.

Après 10 minutes de pose la mèche est séchée pendant 2 minutes au sèche-cheveux.

La mèche obtenue possède un reflet esthétique bleu ou vert selon l'angle d'observation. L'effet observé est rémanent à au moins six shampooings.

Le mode d'application peut également être le suivant :

Une solution dans le milieu cosmétique est préparée avec 10% d'agent de coloration 2. Le monomère est ajouté de manière à ce qu'il ait une concentration finale en monomère de 10%.

0.5g de cette solution est appliqué sur la mèche humidifiée avec 0.5g d'eau.

Après 10 minutes de pose, la mèche est séchée pendant 2 minutes au sèche-cheveux.

### Exemple 3 : monomère méthylheptylcyanoacrylate

La composition suivante est réalisée :

| | |
|---|---|
| DC 1501 Fluid | 40g |
| DC 245 Fluid | 40g |
| Helicone HC Scarabeus | 10g |
| méthylheptylcyanoacrylate de Chemence | 10g |

1g de la composition est appliqué sur une mèche de 1g de cheveux propre et humide. Après 15 minutes de pause, la mèche est séchée au sèche-cheveux pendant 2 minutes. La coloration de la mèche obtenue passe du violet au vert selon l'angle d'observation.

### Exemple 4 : monomère méthylheptylcyanoacrylate avec acide acétique

La composition suivante est réalisée :

| | |
|---|---|
| DC 1501 Fluid | 40g |
| DC 245 Fluid | 39.75g |
| Helicone HC Scarabeus | 10g |
| méthylheptylcyanoacrylate de Chemence | 10g |
| Acide acétique | 0.25g |

1g de la composition est appliqué sur une mèche de 1g de cheveux propre et humide. Après 15 minutes de pause, la mèche est séchée au sèche-cheveux pendant 2 minutes. La coloration de la mèche obtenue passe du violet au vert selon l'angle d'observation.

### Exemple 5 : monomère éthylhexylcyanoacrylate

La composition suivante est réalisée :

| | |
|---|---|
| DC 1501 Fluid | 40g |
| DC 245 Fluid | 40g |
| Helicone HC Scarabeus | 10g |
| Ethylhexylcyanoacrylate de Tong Shen | 10g |

1g de la composition est appliqué sur une mèche de 1g de cheveux propre et humide. Après 15 minutes de pause, la mèche est séchée au sèche-cheveux pendant 2 minutes. La coloration de la mèche obtenue passe du violet au vert selon l'angle d'observation.

### Exemple 6 : monomère butylcyanoacrylate

La composition suivante est réalisée :

| | |
|---|---|
| DC 1501 Fluid | 40g |
| DC 245 Fluid | 39g |
| Helicone HC Scarabeus | 10g |
| Butylcyanoacrylate B-60 de Tong Shen | 10g |
| Acide acétique | 1 g |

1.5g de la composition est appliqué sur une mèche de 1g de cheveux propre et humide. Après 15 minutes de pause, la mèche est séchée au sèche-cheveux pendant 2 minutes. La coloration de la mèche obtenue passe du violet au vert selon l'angle d'observation.

### Exemple 7 : mélange de monomères methylheptylcyanoacrylate et ethylhexylcyanoacrylate

La composition suivante est réalisée :

| | |
|---|---|
| DC 1501 Fluid | 40g |
| DC 245 Fluid | 40g |
| Helicone HC Scarabeus | 10g |
| Methyheptylcyanoacrylate de Chemence | 9g |
| Ethylhexylxyanoacrylate O-60 de Tong Shen | 1 g |

1g de la composition est appliqué sur une mèche de 1g de cheveux propre et humide. Après 15 minutes de pause, la mèche est séchée au sèche-cheveux pendant 2 minutes. La coloration de la mèche obtenue passe du violet au vert selon l'angle d'observation.

### Exemple 8 : mélange de monomères methylheptylcyanoacrylate et butylcyanoacrylate

La composition suivante est réalisée :

| | |
|---|---|
| DC 1501 Fluid | 40g |
| DC 245 Fluid | 40g |
| Helicone HC Scarabeus | 10g |
| méthylheptylcyanoacrylate de Chemence | 7g |
| butylcyanoacrylate B-60 de Tong Shen | 3g |

1g de la composition est appliqué sur une mèche de 1g de cheveux propre et humide. Après 15 minutes de pause, la mèche est séchée au sèche-cheveux pendant 2 minutes. La coloration de la mèche obtenue passe du violet au vert selon l'angle d'observation.

### Exemple 9 : monomère ethoxyethylcyanoacrylate

La composition suivante est réalisée :

| | |
|---|---|
| DC 1501 Fluid | 40g |
| DC 245 Fluid | 35g |
| Helicone HC Scarabeus | 10g |
| ethoxyethylcyanoacrylate EO-460 de Tong Shen | 10g |
| Acide acétique | 5g |

1.5g de la composition est appliqué sur une mèche de 1g de cheveux propre et humide. Après 15 minutes de pause, la mèche est séchée au sèche-cheveux pendant 2 minutes. La coloration de la mèche obtenue passe du violet au vert selon l'angle d'observation.

## Revendications

1. Utilisation pour le traitement des matières kératiniques, et notamment des fibres kératiniques telles que les cheveux, d'une composition comprenant, dans un milieu cosmétiquement acceptable, au moins un monomère électrophile, et au moins un agent cristaux liquides.

2. Utilisation selon la revendication 1, **caractérisée en ce que** le ou les monomères électrophiles répondent à la formule : 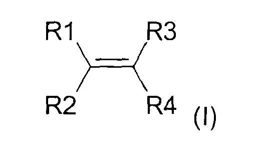 dans laquelle :
R₁ et R₂ désignent chacun, indépendamment l'un de l'autre, un groupe peu ou non électro-attracteur choisi parmi :
- un atome d'hydrogène,
- un groupe hydrocarboné saturé ou non, linéaire, ramifié ou cyclique, comportant de 1 à 20 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
- un résidu polyorganosiloxane modifié ou non,
- un groupement polyoxyalkylène,
R₃ et R₄ désignent chacun, indépendamment l'un de l'autre, un groupe électro-attracteur choisi parmi les groupements -N(R)₃⁺, - S(R)₂⁺, -SH₂⁺, -NH₃⁺, -NO₂, -SO₂R, -C≡N, -COOH, -COOR, -COSR, - CONH₂, -CONHR, -F, -Cl, -Br, -I, -OR, -COR, -SH, -SR, -OH, les groupes alcényle linéaires ou ramifiés, les groupes alcynyle linéaires ou ramifiés, les groupements mono- ou polyfluoroalkyle en C₁- C₄, les groupements aryle et aryloxy,
R désigne un groupe hydrocarboné saturé ou non, linéaire, ramifié ou cyclique, comportant de 1 à 20 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère, R' désignant un radical alkyle en C₁-C₁₀.

3. Utilisation selon la revendication 2, **caractérisée en ce que** le ou les monomères électrophiles sont choisis parmi les composés de formule : 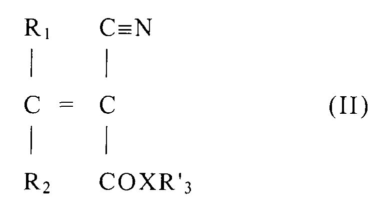 X désignant NH,S,O,
R₁ et R₂ ayant les mêmes significations que précédemment,
R'₃ pouvant désigner un atome d'hydrogène ou un radical R tel que défini dans la revendication 2.

4. Utilisation selon la revendication 3, **caractérisée en ce que** le ou les monomères sont choisis parmi les 2-cyanoacrylates de polyfluoroalkyle en C₁-C₂₀, les cyanoacrylates d'alkyle en (C₁-C₁₀) ou d'(alcoxy en C₁-C₄)(alkyle en C₁-C₁₀).

5. Utilisation selon la revendication 4, **caractérisée en ce que** le ou les monomères sont choisis parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

6. Utilisation selon la revendication 3, **caractérisée en ce que** le ou les monomères électrophiles répondent à la formule (III) : 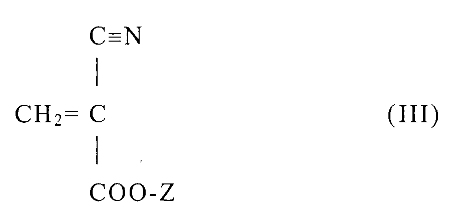 dans laquelle : Z=-(CH₂)₇-CH₃,
-CH(CH₃)-(CH₂)₅-CH₃,
-CH₂-CH(C₂H₅)-(CH₂)₃₋CH₃,
-(CH₂)₅-CH(CH₃)-CH₃,
-(CH₂)₄-CH(C₂H₅)-CH₃.

7. Utilisation selon l'une quelconque des revendications précédentes, **caractérisée en ce que** le monomère est présent dans la composition à des teneurs comprises entre 0,001 et 80% en poids, de préférence entre 0,1 et 40%, et encore de préférence entre 1 et 20% en poids du poids total de la composition.

8. Utilisation selon l'une quelconque des revendications précédentes, **caractérisée en ce que** les monomères sont fixés de façon covalente sur des supports tels que des polymères, des oligomères ou des dendrimères.

9. Utilisation selon l'une quelconque des revendications précédentes, **caractérisée en ce que** le milieu cosmétiquement acceptable est anhydre.

10. Utilisation selon la revendication 9, **caractérisé en ce que** le milieu cosmétiquement acceptable est choisi parmi les huiles organiques, les silicones, les huiles minérales, les huiles végétales, les cires, les alcanes en C₅-C₁₀, l'acétone, la méthyléthylcétone, les esters d'acides en C₁-C₂₀ et d'alcools en C₁-C₈, le diméthoxyéthane, le diéthoxyéthane, les alcools gras en C₁₀-C₃₀, les acides gras en C₁₀-C₃₀, les amides gras en C₁₀-C₃₀, les esters d'alcool gras en C₁₀-C₃₀ et leurs mélanges.

11. Utilisation d'une composition selon l'une quelconque des revendications précédentes, **caractérisée en ce que** la composition comprend des inhibiteurs de polymérisation.

12. Utilisation selon la revendication 11, **caractérisée en ce que** les inhibiteurs sont des inhibiteurs de polymérisation anioniques et/ou radicalaires.

13. Utilisation selon la revendication 11, **caractérisée en ce que** les inhibiteurs de polymérisation sont choisis parmi le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène et leurs mélanges.

14. Utilisation selon l'une quelconque des revendications 11 à 13, **caractérisée en ce que** les inhibiteurs de polymérisation sont présents en des quantités allant de 10 ppm à 20%, de préférence allant de 10 ppm à 5%, et plus préférentiellement entre 10 ppm et 1% en poids du poids total de la composition.

15. Utilisation selon l'une quelconque des revendications précédentes, **caractérisée en ce que** l'agent cristaux liquides est choisi parmi :
- les composés à fonction cholestérique, dont la structure est la suivante : 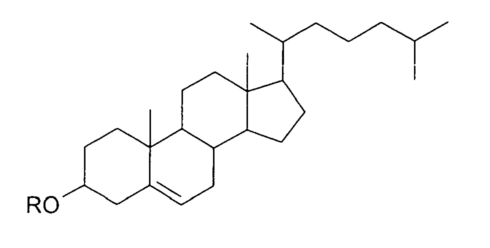 R est un groupement alkyl, alkylcarbonyl comprenant de 1 à 30 atomes de carbone substitué ou non par des groupements cycliques, aromatiques, halogènes, ramifié ou non,
- les composés définis par la formule suivante :
A₁^{5'}-[(X¹)ₐ- (A¹)_{b}- (A²)_{c}]_{d}-Zₑ-[(X²)_{f}-(A³)_{g}-(A⁴)ₕ]ᵢ-Aₖ⁵
dans laquelle
X¹ et X² représentent indépendamment des radicaux divalents - O-, -COO-, -CONH-, -CO-, -S-, -C≡C-, -CH=CH-, -CH₂-CH₂,-, -CH=N-, -N=N- ou -N=N(O)-, -CH=N-N=CH-, -CH=CH-COO-, -OCO-CH=CH-
A¹, A², A³ et A⁴, représentent indépendamment des radicaux divalents 1,4-phénylène, 1,4-cyclohexylène, arylènes, hétéroarylènes, hétérocycloalkylènes, cycloalkylènes éventuellement substitués,
Z représente indépendamment des radicaux divalents à tétravalents, benzène-1,4 cyclohexane ou benzène-1,3-cyclopentane,
A⁵ et A^{5'} représentent indépendamment un radical alkyle, alkoxy ou cycloalkyle, saturé ou insaturé, ayant 1 à 16 atomes de carbone, un radical stéroïdien, un halogène, un atome d'hydrogène, un radical hydroxyle, nitrile ou trialkylsilyloxy.
a, b, c, d, f, g, h, i k et l représentent indépendamment un entier allant de 0 à 3,
e représente 0 ou 1,
avec la somme a+b+c+d+e+f+g+h+i+k étant supérieure ou égale à 2, et la somme d+i étant inférieure ou égale à 4,
étant entendu que ce groupe ne comprend pas de radical peroxyde,
- un polymère obtenu par la polymérisation de monomères mésogéniques de structure suivante :
Y¹-B¹-M-R
dans laquelle :
Y¹ représente un groupe polymérisable choisi parmi les groupements acrylate ou méthacrylate, un groupement époxy, isocyanante, hydroxy, vinyléther (-O-CH=CH2), vinylester (-CO-O-C=CH2), styryl, trialkoxysiloxy
B^{l} représente un groupement de formule -CₙH₂ₙ-, dans lequel n est un nombre entier allant de 0 à 20 et un ou plusieurs groupes méthylènes dudit groupement -CₙH₂ₙ- pouvant être substitués par un atome d'halogène ou être remplacé (lorsque les méthylènes ne sont pas adjacents) par un ou plusieurs groupes suivants : -O-, -NH-, -OCO-, - OCO-O-, -S-CO--COO-, -CONH-, -CO-, -S-, -C≡C-, -CH=CH-.
M représente un groupement de formule :
-[(X¹)ₐ- (A¹)_{b}- (A²)_{c}]_{d}-Zₑ-[(X²)_{f}-(A³)_{g}-(A⁴)ₕ]ᵢ-
dans laquelle
X¹ et X² représentent indépendamment des radicaux divalents - O-, -COO-, -CONH-, -CO-, -S-, -C≡C-, -CH=CH-, -CH₂-CH₂,-, -CH=N-, -N=N- ou -N=N(O)-, -CH=N-N=CH-, -CH=CH-COO-, -OCO-CH=CH-
A¹, A², A³ et A⁴, représentent indépendamment des radicaux divalents 1,4-phénylène, 1,4-cyclohexylène, arylènes, hétéroarylènes, cycloalkylènes éventuellement substitués,hétérocycloalkylènes
Z représente indépendamment des radicaux divalents à tétravalents, benzène-1,4 cyclohexane ou benzène-1,3-cyclopentane, Z peut aussi représenter un groupement chiral divalent comprenant au moins 4 atomes de carbone, notamment de 4 à 20 atomes de carbone et mieux de 4 à 10 atomes de carbone, le groupement chiral divalent comprenant au moins un carbone asymétrique.

16. Utilisation selon la revendication 15, **caractérisée en ce que** l'agent cristaux liquides est choisi parmi le cholesterol erucyl carbonate, le cholesterol methyl carbonate, le cholesterol oleyl carbonate, le cholesterol para-nonyl phneyl carbonate, le cholesterol phenyl carbonate, la cholesterol acetate, le cholesterol benzoate, le cholesterol butyrate, le cholesterol isobutyrate, le cholesterol chloride, le cholesterol chloroacetate, le cholesterol cinnamate, le cholesterol crotanoate, le cholesterol decanoate, le cholesterol erucate, le cholesterol heptanoate, le cholesterol hexanoate, le cholesterol myristate, le cholesterol nonaoate, le cholesterol octanoate, le cholesterol oleate, le cholesterolpropionate, le cholesterol valerate, le dicholesteryl carbonate.

17. Utilisation selon la revendication 15, **caractérisée en ce que** l'agent cristaux liquides est le Polyacrylate-4.

18. Utilisation selon l'une quelconque des revendications 1 à 14, **caractérisée en ce que** l'agent cristaux liquides est choisi parmi les polyorganosiloxanes cycliques greffés par des groupements choléstériques et biphényliques.

19. Utilisation selon l'une quelconque des revendications précédentes, **caractérisée en ce que** l'agent cristaux liquides est présent dans la composition à des teneurs comprises entre 0,05 et 40% en poids, de préférence entre 0,1 et 35%, et encore de préférence entre 0,25 et 25% en poids du poids total de la composition.

20. Utilisation selon l'une quelconque des revendications précédentes, **caractérisée en ce que** la composition comprend en outre au moins un agent choisi parmi les agents réducteurs, les corps gras, les plastifiants, les adoucissants, les agents anti-mousse, les agents hydratants, les pigments, les argiles, les charges minérales, les filtres UV, les colloïdes minéraux, les peptisants, les solubilisants, les parfums, les conservateurs, les tensioactifs anioniques, cationiques, non ioniques ou amphotères, les polymères fixants ou non, les polyols, les protéines, les vitamines, les colorants directs ou d'oxydation, et les agents nacrants.

21. Utilisation selon l'une quelconque des revendications précédentes, **caractérisée en ce que** la composition est sous forme de lotion, de spray ou de mousse.

22. Composition cosmétique, **caractérisée en ce qu'**elle comprend, dans un milieu cosmétiquement acceptable, au moins un monomère électrophile, et au moins un agent cristaux liquides différent des agents cristaux liquides cholestériques.

23. Composition selon la revendication 22, **caractérisée en ce que** le monomère électrophile est choisi parmi les monomères définis dans les revendications 2 à 6.

24. Composition selon la revendication 22 ou 23, **caractérisée en ce que** l'agent cristaux liquides est choisi parmi les composés suivants :
- les composés définis par la formule suivante :
A₁^{5'}- [(X¹)ₐ- (A¹)_{b}- (A²)_{c}]_{d}-Zₑ-[(X²)_{f}-(A³)_{g}-(A⁴)ₕ]ᵢ-Aₖ⁵
dans laquelle
X¹ et X² représentent indépendamment des radicaux divalents - O-, -COO-, -CONH-, -CO-, -S-, -C≡C-, -CH=CH-, -CH₂-CH₂,-, -CH=N-, -N=N- ou -N=N(O)-, -CH=N-N=CH-, -CH=CH-COO-, -OCO-CH=CH-
A¹, A², A³ et A⁴, représentent indépendamment des radicaux divalents 1,4-phénylène, 1,4-cyclohexylène, arylènes, hétéroarylènes, hétérocycloalkylènes, cycloalkylènes éventuellement substitués,
Z représente indépendamment des radicaux divalents à tétravalents, benzène-1,4 cyclohexane ou benzène-1,3-cyclopentane,
A⁵ et A^{5'} représentent indépendamment un radical alkyle, alkoxy ou cycloalkyle, saturé ou insaturé, ayant 1 à 16 atomes de carbone, un radical stéroïdien, un halogène, un atome d'hydrogène, un radical hydroxyle, nitrile ou trialkylsilyloxy.
a, b, c, d, f, g, h, i k et l représentent indépendamment un entier allant de 0 à 3,
e représente 0 ou 1,
avec la somme a+b+c+d+e+f+g+h+i+k étant supérieure ou égale à 2, et la somme d+i étant inférieure ou égale à 4,
étant entendu que ce groupe ne comprend pas de radical peroxyde,
- le polymère obtenu par la polymérisation de monomères mésogéniques de structure suivante :
Y'-B'-M-R
dans laquelle :
Y^{l} représente un groupe polymérisable choisi parmi les groupements acrylate ou méthacrylate, un groupement époxy, isocyanante, hydroxy, vinyléther (-O-CH=CH₂), vinylester (-CO-O-C=CH₂), styryl, trialkoxysiloxy
B¹ représente un groupement de formule -CₙH₂ₙ-, dans lequel n est un nombre entier allant de 0 à 20 et un ou plusieurs groupes méthylènes dudit groupement -CₙH₂ₙ- pouvant substitués par un atome d'halogène ou être remplacé (lorsque les méthylènes ne sont pas adjacents) par un ou plusieurs groupes suivants : -O-, -NH-, -OCO-, -OCO-O-, -S-CO-COO-, -CONH-, -CO-, -S-, -C≡C-, -CH=CH-.
M représente un groupement de formule :
-[(X¹)ₐ- (A¹)_{b}- (A²)_{c}]_{d}-Zₑ-[(X²)_{f}-(A³)_{g}-(A⁴)ₕ]ᵢ-
dans laquelle
X¹ et X² représentent indépendamment des radicaux divalents - O-, -COO-, -CONH-, -CO-, -S-, -C≡C-, -CH=CH-, -CH₂-CH₂,-, -CH=N-, -N=N- ou -N=N(O)-, -CH=N-N=CH-, -CH=CH-COO-, -OCO-CH=CH-
A¹, A², A³ et A⁴, représentent indépendamment des radicaux divalents 1,4-phénylène, 1,4-cyclohexylène, arylènes, hétéroarylènes, cycloalkylènes éventuellement substitués,hétérocycloalkylènes
Z représente indépendamment des radicaux divalents à tétravalents, benzène-1,4 cyclohexane ou benzène-1,3-cyclopentane, Z peut aussi représenter un groupement chiral divalent comprenant au moins 4 atomes de carbone, notamment de 4 à 20 atomes de carbone et mieux de 4 à 10 atomes de carbone, le groupement chiral divalent comprenant au moins un carbone asymétrique,

25. Composition selon la revendication 24, **caractérisée en ce que** l'agent cristaux liquides est le Polyacrylate-4.

26. Composition selon la revendication 22, **caractérisée en ce que** l'agent cristaux liquides est choisi parmi les polyorganosiloxanes cycliques greffés par des groupements choléstériques et biphényliques.

27. Procédé de traitement des matières kératiniques, **caractérisé en ce qu'**il comprend une étape d'application sur les matières kératiniques d'au moins un agent cristaux liquides et une étape d'application sur les matières kératiniques d'au moins un monomère électrophile.

28. Procédé selon la revendication 27, **caractérisé en ce que** l'application du ou des agents cristaux liquides est réalisée avant l'application du ou des monomères électrophiles.

29. Procédé de traitement des matières kératiniques, **caractérisé en ce qu'**on applique sur les matières kératiniques une composition utilisée dans l'une quelconque des revendications 1 à 21, en présence d'un agent nucléophile.

30. Procédé selon la revendication 29, **caractérisé en ce que** l'agent nucléophile est choisi parmi les composés moléculaires, les oligomères, les dendrimères ou polymères possédant des fonctions nucléophiles choisies parmi: R2N-, NH2-, Ph3C-, R3C-, PhNH-, pyridine, ArS-, R-C≡C-, RS-, SH, RO-, R2NH, ArO-, N3-, OH-, ArNH2, NH3, I-, Br-, Cl-, RCOO-, SCN-, ROH, RSH, NCO-, CN-, NO3-, ClO4- et H2O, Ph représentant le groupe phényle ; Ar représentant un groupe aryle et R représentant un groupe alkyle en Cl-C10.

31. Procédé selon la revendication 29, **caractérisé en ce que** l'agent nucléophile est l'eau.

32. Procédé selon l'une quelconque des revendications 29 à 31, **caractérisé en ce que** l'on applique la composition sur les matières kératiniques préalablement humidifiées à l'aide d'une solution aqueuse dont le pH a été ajusté à l'aide d'une base, d'un acide ou d'un mélange acide/base.

33. Procédé selon l'une quelconque des revendications 28 à 32, **caractérisé en ce que** les matières kératiniques sont pré-imprégnées à l'aide d'un agent nucléophile autre que l'eau.

34. Procédé selon l'une quelconque des revendications 29 à 31, **caractérisé en ce que** les matières kératiniques sont préalablement réduites avant application de la composition.

35. Procédé selon la revendication 34, **caractérisé en ce que** l'agent réducteur est choisi parmi le thiosulfate de sodium anhydre, le métabisulfite de sodium en poudre, la thiourée, le sulfite d'ammonium, l'acide thioglycolique, l'acide tiolactique, le thiolactate d'ammonium, le mono-thioglycolate de glycérol, le thioglycolate d'ammonium, le thioglycérol, l'acide 2,5-dihydroxybenzoique, le di-thioglycolate de diammonium, le thioglycolate de strontium, le thioglycolate de calcium, le formo-sulfoxylate de zinc, le thioglycolate d'isooctyle, la di-cystéine, le thioglycolate de monoéthanolamine.

36. Procédé selon l'une des revendications 29 à 35, **caractérisé en ce que** la composition comprend en outre un polymère choisi parmi le poly(méthacrylate de méthyle) et les copolymères à base de cyanoacrylates.

37. Procédé selon l'une des revendications 28 à 36, **caractérisé en ce que** l'application de la composition est suivie d'un rinçage.

38. Kit comprenant une première composition contenant au moins un monomère électrophile et éventuellement au moins un inhibiteur de polymérisation anionique et/ou radicalaire, ainsi qu'une deuxième composition comprenant dans un milieu cosmétiquement acceptable au moins un agent cristaux liquides.
